# EUROPEAN PATENT APPLICATION

(11) **EP 4 692 992 A1**
(43) Date of publication of application: **11.02.2026**
(21) Application number: 24784416.0
(22) Date of filing: 03.04.2024
(51) Int. Cl.: G06F 3/01, A61B 5/16

(54) **BRAIN COMPUTER SIGNAL SECURITY SYSTEM AND USE METHOD THEREOF**

(30) Priority: 04.04.2023 CN 202310358178
(71) Applicant: Cao, Qingheng, Beijing 100022 (CN)
(72) Inventor: Cao, Qingheng, Beijing 100022 (CN)
(74) Representative: Danubia Patent & Law Office LLC
(86) International application number: PCT/CN2024/086128
(87) International publication number: WO 2024/208334

(57) **Abstract**

The present invention discloses a braincomputer signal safety system and its method of use, which pertains to the technical field of intelligent information processing. The braincomputer signal safety system comprises:
An information acquisition module (consistent with the "data acquisition module" elsewhere in this specification) configured to acquire braincomputer signals and safety information related to the braincomputer signals;
An analysis module configured to take the braincomputer signals and the safety information of the braincomputer signals as inputs, conduct analysis using a braincomputer signal safety model, determine whether the braincomputer signals meet the safety constraints, and obtain appropriate braincomputer signal safety measures when the safety constraints are met;
A safety module configured to perform safety processing on the braincomputer signals according to the appropriate braincomputer signal safety measures.

By performing safety processing on the braincomputer signals through the appropriate braincomputer signal safety measures derived from the preestablished braincomputer signal safety model, the safety of the BrainComputer Interface (BCI) can be improved, the usage effect of the BCI can be enhanced, and safety hazards to the subjects affected by the braincomputer signals can be avoided.

## Description

This application claims priority to Chinese Patent Application No. 202310358178.2, titled "A Brain-Computer Signal Safety System and Its Usage Method", filed with the China National Intellectual Property Administration on April 4, 2023. The entire content of the aforementioned application is incorporated herein by reference.

### Technical Field

The present invention pertains to the technical field of intelligent information processing, and more particularly, to a brain-computer signal safety system and its method of use.

### Background Art

A Brain-Computer Interface (BCI) establishes a direct communication pathway between the brain (human or animal) and external devices, enabling information exchange. BCI technology shows significant promise in restoring impaired functions such as hearing, vision, and limb movement, allowing control of prosthetic devices akin to natural limbs. With advancing technology and knowledge, BCI research is evolving from merely restoring function to enhancing human capabilities. The continued development of BCI holds great potential for improving quality of life.

BCls are primarily categorized into implantable and non-implantable types. Implantable BCls involve surgically placing devices inside the body to interface directly with the brain or nervous tissue. Non-implantable BCls typically use external attachments, such as patches, to stimulate the brain or nerves through methods like electrical currents. Given that BCI interacts directly with the brain, stringent safety requirements are paramount.

Current BCls lack a unified standard for brain-computer signal safety. Typically, a safety range is defined, and signals are monitored for compliance. Deviations trigger alerts and potential safety measures. However, the accuracy and precision of these existing safety measures are often insufficient, potentially causing harm to the subjects affected by the brain-computer signals.

### Summary of the Invention

The objective of the present invention is to provide a brain-computer signal safety system and its method of use. This system aims to enhance the safety of BCI, improve its effectiveness, and prevent safety hazards to the subjects (i.e., the human bodies) impacted by the brain-computer signals.

To achieve this objective, the present invention provides the following solutions:
A brain-computer signal safety system comprises: a database module, a data acquisition module, an analysis module, and a safety module.

The database module is configured to store a BCI database. The BCI database includes a brain-computer signal safety model. This model incorporates safety constraints required for brain-computer signals, which vary based on different signal parameters, information sources, information channels, access methods, modes, frequencies, intensities, durations, intervals, information volumes, and the subjects receiving or affected by the signals.

The data acquisition module (also referred to as the "information acquisition module" elsewhere in this specification) is configured to acquire brain-computer signals and their associated safety information.

The analysis module is configured to analyze the acquired brain-computer signals, their safety information, and the brain-computer signal safety model to determine appropriate brain-computer signal safety measures.

The safety module is configured to perform safety processing on the brain-computer signals according to the determined safety measures, thereby enhancing signal safety.

A method for using the aforementioned brain-computer signal safety system comprises the following steps:
1. Establishing a BCI database containing a brain-computer signal safety model, which includes safety constraints for brain-computer signals based on different parameters as mentioned above.
2. Acquiring brain-computer signals and their safety information.
3. Analyzing the signals, safety information, and the safety model to obtain suitable safety measures.
4. Performing safety processing on the signals based on these measures to improve safety.

In another aspect, a brain-computer signal safety system comprises:
An information acquisition module (consistent with the "data acquisition module" above) for acquiring brain-computer signals and their safety information. The brain-computer signals include attributes such as mode, type, form, content, function, target neurons, information volume, signal intensity, frequency, purpose, target subject (consistent with "objects affected by brain-computer signals"), access method, and location. The safety information pertains to the safety constraints required for these signals.

An analysis module that takes the brain-computer signals and their safety information as inputs, utilizes the brain-computer signal safety model for analysis, determines whether the signals meet the safety constraints, and obtains suitable safety measures when constraints are met. The safety model includes constraints and corresponding safety measures for signals varying in the aforementioned parameters, as well as information forms and contents.

A safety module that performs safety processing on the brain-computer signals according to the suitable safety measures.

In some embodiments, the system further includes a database module storing a BCI database. This database contains personalized brain-computer signal safety models for different user types or individual users. These models are established by considering the potential impacts of brain-computer signals (with different parameters) on users, combined with at least one type of information about the target subject. This subject information includes demographics, genetic factors, physiological factors, psychological factors, physical factors, living status/life history, exercise status, work/rest status, learning status, work status, disease status, medication history, medical device usage, diet, physiological/psychological trauma, surgical history, radiation exposure, physical therapy, rehabilitation, operations, examinations, and psychological interventions.

The analysis module is further configured to select a suitable brain-computer signal safety model from the database based on the acquired signals and safety information, before performing the analysis. The suitable model may be a pre-existing model from the database or an adaptation thereof based on the actual signal characteristics.

In some embodiments, when determining suitable safety measures, the analysis module also considers interactions between different dimensions of a single brain-computer signal or between multiple signals. These interactions may include signal enhancement or attenuation, changes in information form or content, effects on reception channels/sites, and alterations in overall effects.

In some embodiments, the analysis module assigns levels or scores to different potential safety measures based on analysis items such as risk, harmfulness, complexity, constraints, effectiveness, time requirement, cost, convenience, economy, information integrity/authenticity/accuracy, user benefit, and user damage. Based on the scores for each item and the safety model, a comprehensive score is calculated for each safety measure. The suitable safety measure is then determined according to this comprehensive score.

In some embodiments, the brain-computer signal safety model further includes safety constraints and corresponding measures for brain-computer signals with different contents, functions, and effects. These effects encompass impacts on knowledge, cognition, values, interests, methods, goals, emotions, states, physical fitness, skills, behaviors, intelligence, memory, physiological functions, expression, immunity, excitement level, and the establishment/disconnection of brain synapses/connections.

In some embodiments, the system further includes:
A processing module configured to perform operations such as translation, matching, conversion, and recognition on the brain-computer signals to extract characteristic information. Based on this characteristic information and the suitable safety measures, the module preprocesses the signals before final safety processing to ensure signal functionality.

An adjustment and optimization module that acquires the actual effect of the applied safety processing, analyzes this effect, and uses the analysis result to adjust and optimize the brain-computer signal safety model.

A signal adjustment module that adjusts the brain-computer signals by applying measures such as deletion, reduction, decrease, adjustment, change, translation, conversion, reminder, warning, shutdown, enhancement, and increase.

### Technical Effects

According to specific embodiments provided by the present invention, the following technical effects are disclosed:
The invention provides a brain-computer signal safety system and its method of use. The system acquires brain-computer signals and their safety information, analyzes them using a pre-established safety model to determine if safety constraints are met and to obtain suitable safety measures, and then performs safety processing accordingly. This process enhances BCI safety, improves BCI effectiveness, and prevents harm to subjects affected by the signals.

### Brief Description of the Drawings

To illustrate the embodiments of the present invention or the technical solutions in the prior art more clearly, the drawings required for describing the embodiments are briefly introduced below. Obviously, the drawings in the following description represent only some embodiments of the present invention. For those skilled in the art, other drawings can be derived from these without creative effort.

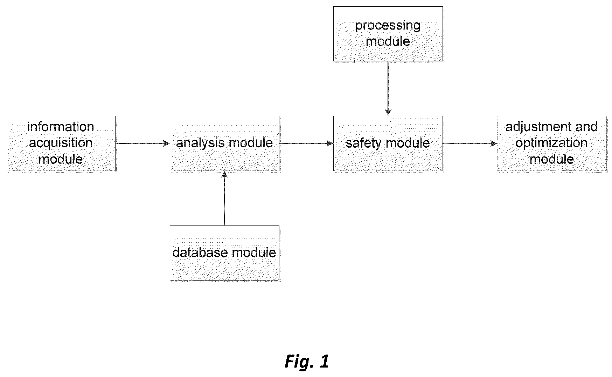

Figure 1 is a system block diagram of a brain-computer signal safety system provided in Embodiment 3 of the present invention.

### Detailed Description of the Embodiments

The technical solutions in the embodiments of the present invention will be clearly and completely described below with reference to the accompanying drawings. Obviously, the described embodiments are merely a part, not all, of the embodiments of the present invention. Based on the embodiments herein, all other embodiments obtained by persons of ordinary skill in the art without creative effort shall fall within the protection scope of the present invention.

The objective of the present invention is to provide a brain-computer signal safety system and its method of use, which can improve BCI safety, enhance its effectiveness, and avoid causing harm to subjects affected by brain-computer signals. To make the objectives, features, and advantages of the present invention more apparent and comprehensible, the invention is described further in detail below with reference to the drawings and specific embodiments.

### Embodiment 1

A brain-computer signal safety system comprises: a database module, a data acquisition module, an analysis module, and a safety module.

The database module is configured to store a BCI database containing a brain-computer signal safety model. The model includes safety constraints required for brain-computer signals corresponding to different signal parameters, information sources, information channels, access methods, modes, frequencies, intensities, durations, intervals, information volumes, and target subjects.

The data acquisition module is configured to acquire brain-computer signals and their safety information.

The analysis module is configured to analyze the signals, safety information, and the safety model to obtain suitable safety measures.

The safety module is configured to perform safety processing on the signals based on the suitable safety measures to enhance safety.

### Embodiment 2

A method for using the brain-computer signal safety system described in Embodiment 1 comprises:
1. Establishing a BCI database containing the brain-computer signal safety model with constraints based on various signal and subject parameters.
2. Acquiring brain-computer signals and their safety information.
3. Analyzing the signals, safety information, and model to determine suitable safety measures.
4. Performing safety processing on the signals according to these measures.

### Embodiment 3

As shown in Figure 1, a brain-computer signal safety system includes:
An information acquisition module for acquiring brain-computer signals and their safety information. The signals are characterized by mode, type, form, content, function, target neurons, information volume, intensity, frequency, purpose, target subject, access method, and location. The safety information relates to the required safety constraints for these signals.

An analysis module that uses the brain-computer signals and safety information as inputs, applies the brain-computer signal safety model to check constraint compliance, and obtains suitable safety measures when constraints are met. The safety model contains constraints and measures for signals varying in the listed parameters, including information forms and contents.

A safety module that performs safety processing based on the suitable safety measures.

This system is applicable to both implantable and non-implantable brain-computer signals.

The system further includes a database module storing a BCI database with personalized brain-computer signal safety models for user types or individuals. These models are built by assessing potential impacts of signals (with different parameters) on users (affecting health, behavior, emotions, cognition, physiology, etc.), considering also subject information like demographics, genetics, physiology, psychology, lifestyle, medical history, etc., as listed previously.

In this embodiment, the BCI database is established first, containing the safety model. Preferably, the model also includes constraints and measures for signals with different contents, functions, and effects (e.g., impacts on knowledge, values, skills, memory, etc.). For instance, an atheism-related signal might minimally affect atheist users but significantly impact others, posing potential risks.

Since the brain is functionally organized (e.g., motor cortex for movement, sensory cortex for perception), brain-computer signals vary in mode, target neurons, intensity, frequency, and information volume based on their purpose. Thus, establishing a safety model is necessary. The model defines safety constraints and measures by evaluating potential impacts of signal parameters (and optionally content/function/effects) on users. Modeling methods include manual setting, Al/machine learning, and big data analysis. Impacts can be physiological, psychological, etc., some potentially harmful, necessitating constraints (e.g., limiting voltage, current, frequency for electrical signals).

Based on this, a general safety model is established. The analysis module uses the acquired signals and safety information as input to this model for analysis.

Due to individual differences, the safety model can be personalized based on subject information (demographics, genetics, physiology, etc.), making it applicable to different subjects.

Factors influencing safety constraints may include: population factors (nationality, race, gender, age); genetic factors (genes, mutations, defects, family history); physiological factors (fitness, nutrition, sensory abilities); psychological factors (mental health, emotions, intelligence); physical factors (height, weight, strength); living status/life history (work, diet, exercise); disease factors; time factors (time of day, season); environmental factors (temperature, humidity, altitude, radiation); and other factors like medication, medical devices, diet, trauma, surgery, therapy, etc.

The information acquisition module gathers brain-computer signals from BCI or related systems, including the listed attributes. Safety information encompasses subject's basic info, genetic data, medical history (allergies, medications, surgery), lifestyle status (learning, work, exercise, sleep), environmental data, and various medical indicators (vital signs, blood tests, imaging results, psychological assessments). This information can be sourced from personal databases, health records, electronic medical records, wearable devices, sensors, social platforms, or big data analysis. Missing data can be provided by users or prompted for collection.

The processing module can analyze signal characteristics using time-domain methods (zero-crossing, histogram, peak detection, wavelet transform), frequency-domain methods (power spectrum estimation, FFT, AAR model, wavelet transform), or time-frequency methods (Wigner distribution, wavelet transform) to handle non-stationary signals.

The technical features of the above embodiments can be freely combined. For brevity, not all combinations are described, but any conflict-free combination falls within the scope of this specification.

The principles and implementation methods of the present invention are explained herein using specific examples. The description of the embodiments is intended only to aid in understanding the method and core concepts of the invention. Meanwhile, for those skilled in the art, modifications in specific implementation methods and application scopes may occur based on the ideas of the present invention. In summary, the content of this specification should not be construed as limiting the invention.

## Claims

1. A brain-computer signal safety system, comprising:
- A database module configured to store a Brain-Computer Interface (BCI) database, wherein the BCI database contains a brain-computer signal safety model, and the brain-computer signal safety model includes safety constraints required for brain-computer signals corresponding to different signals, information sources, information channels, access methods, modes, frequencies, intensities, durations, intervals, information amounts, subjects receiving/affected by the signals, and combinations thereof;
- A data acquisition module configured to acquire brain-computer signals and safety information related to the brain-computer signals;
- An analysis module configured to conduct analysis by using the brain-computer signals, the safety information related to the brain-computer signals and the brain-computer signal safety model to obtain suitable brain-computer signal safety measures;
- A safety module configured to perform safety processing on the brain-computer signals according to the suitable brain-computer signal safety measures to improve the safety of the brain-computer signals.

2. A method for using the brain-computer signal safety system according to Claim 1, comprising the following steps:
1. Establishing a BCI database containing a brain-computer signal safety model, wherein the brain-computer signal safety model includes safety constraints required for brain-computer signals corresponding to different signals, information sources, information channels, access methods, modes, frequencies, intensities, durations, intervals, information amounts, subjects receiving/affected by the signals, and combinations thereof;
2. Acquiring brain-computer signals and safety information related to the brain-computer signals;
3. Conducting analysis by using the brain-computer signals, the safety information related to the brain-computer signals and the brain-computer signal safety model to obtain suitable brain-computer signal safety measures;
4. Performing safety processing on the brain-computer signals according to the suitable brain-computer signal safety measures to improve the safety of the brain-computer signals.

3. A brain-computer signal safety system, comprising:
- An information acquisition module (consistent with the "data acquisition module" elsewhere in this specification) configured to acquire brain-computer signals and safety information of the brain-computer signals; wherein the brain-computer signals include parameters such as modes, types, forms, contents, functions, target neurons, information amounts, signal intensities, frequencies, usages, target subjects (consistent with "subjects affected by brain-computer signals"), access methods, and positions; and the safety information of the brain-computer signals refers to information related to the safety constraints required for the brain-computer signals;
- An analysis module configured to take the brain-computer signals and the safety information of the brain-computer signals as inputs, conduct analysis by using a brain-computer signal safety model to determine whether the brain-computer signals meet the safety constraints, and obtain suitable brain-computer signal safety measures when the brain-computer signals meet the safety constraints; wherein the brain-computer signal safety model includes safety constraints corresponding to the parameters of the brain-computer signals and brain-computer signal safety measures required when the safety constraints are met;
- A safety module configured to perform safety processing on the brain-computer signals according to the suitable brain-computer signal safety measures.

4. The brain-computer signal safety system according to Claim 3, further comprising a database module configured to store a BCI database containing brain-computer signal safety models for each user type or each individual user; wherein the brain-computer signal safety model for each user type or each individual user is established by evaluating potential impacts of brain-computer signals on users, in combination with at least one type of information about the target subject selected from the group consisting of demographics, genetic factors, physiological factors, psychological interventions, physical factors, living status, disease conditions, interests, habits, and environmental factors, and combinations thereof;
The analysis module is further configured to determine a suitable brain-computer signal safety model according to the acquired brain-computer signals and the safety information of the brain-computer signals before conducting analysis; and the suitable brain-computer signal safety model is either one of the brain-computer signal safety models in the BCI database or a model obtained by adjusting one of the brain-computer signal safety models in the BCI database according to the actual conditions of the brain-computer signals.

5. The brain-computer signal safety system according to Claim 3, wherein when the analysis module acquires the suitable brain-computer signal safety measures, it is further necessary to consider interactions between different dimensions of the brain-computer signals or between more than one brain-computer signal.

6. The brain-computer signal safety system according to Claim 3, wherein the analysis module is further configured to: assign corresponding levels or scores for at least one analysis item selected from risk, harmfulness, complexity, constraints, effectiveness, user damage, time requirement, cost, convenience, economy, information integrity, information authenticity, information accuracy, and user benefit of the brain-computer signal safety measures; calculate a comprehensive level or score of the brain-computer signal safety measures according to the levels or scores of each analysis item and the brain-computer signal safety model; and determine the suitable brain-computer signal safety measures based on said comprehensive level or score.

7. The brain-computer signal safety system according to Claim 3, wherein the brain-computer signal safety model further includes safety constraints required for brain-computer signals with different contents, functions, and effects, and brain-computer signal safety measures required when the safety constraints are met; and the effects of the brain-computer signals include impacts on at least one of knowledge, cognition, values, emotions, behaviors, physiological states, the establishment/disconnection of brain synapses/connections, interests, methods, goals, physical fitness, skills, intelligence, memory, expression, immunity, and excitement level of the target subjects, including but not limited to the foregoing.

8. The brain-computer signal safety system according to Claim 3, further comprising a processing module configured to perform at least one operation among translation, matching, conversion, and recognition on the brain-computer signals to obtain characteristic information of the brain-computer signals; and the processing module is further configured to preprocess the brain-computer signals prior to said safety processing by the safety module according to the characteristic information and the suitable brain-computer signal safety measures to ensure functionality of the brain-computer signals.

9. The brain-computer signal safety system according to Claim 3, further comprising an adjustment and optimization module configured to acquire the actual effect of the safety processing performed by the safety module on the brain-computer signals, conduct analysis on the actual effect to obtain an analysis result, and adjust and optimize the brain-computer signal safety model according to the analysis result.

10. The brain-computer signal safety system according to Claim 3, further comprising a signal adjustment module configured to adjust the brain-computer signals by applying at least one measure selected from deletion, reduction, decrease, adjustment, change, translation, conversion, reminder, warning, shutdown, enhancement, and increase.
